# EUROPEAN PATENT APPLICATION

(11) **EP 3 108 805 A1**
(43) Date of publication of application: **28.12.2016**
(21) Application number: 16174778.7
(22) Date of filing: 16.06.2016
(51) Int. Cl.: A61B 5/00, A61B 5/021, A61B 5/024, A61B 5/0402, A61B 5/0456, A61B 5/11

(54) **TOUCH PANEL APPARATUS FOR MEASURING BIOSIGNALS AND METHOD OF MEASURING PULSE TRANSIT TIME USING THE SAME**

(30) Priority: 23.06.2015 KR 20150089089
(71) Applicant: Samsung Electronics Co., Ltd., Gyeonggi-do 16677 (KR)
(72) Inventor: KIM, Sunkwon, 16678 Gyeonggi-do (KR); KANG, Jaemin, 16678 Gyeonggi-do (KR); KWON, Yongjoo, 16678 Gyeonggi-do (KR)
(74) Representative: Grünecker Patent- und Rechtsanwälte PartG mbB

(57) **Abstract**

A touch panel apparatus and a method are provided. The touch panel apparatus includes a touch panel, a pulse sensor, and a processor. The touch panel detects a heartbeat-based signal from a first part of a target body. The pulse sensor detects a pulse signal from a second part of the target body. The processor acquires a pulse transit time (PTT) based on the detected heartbeat-based signal and the detected pulse signal. The touch panel apparatus contemporaneously detects the heartbeat-based signal and the pulse signal using the touch panel and the pulse sensor, respectively.

## Description

### BACKGROUND

### 1. Field

Apparatuses, devices, methods, and articles of manufacture consistent with the present disclosure relate to a touch panel apparatus for measuring biosignals and a method of measuring a pulse transit time (PTT) using the touch panel apparatus.

### 2. Description of the Related Art

A pulse transit time (PTT) is the time taken for a pulse to move between pulsation positions in two arteries. In general, because the PTT may be measured using biosignals detected from a heart and an end portion of a body, the PTT is the time taken from contraction of the heart until the pulse reaches a hand. The PTT is associated with biological information such as blood pressure.

### SUMMARY

It is an aspect to provide a touch panel apparatus and a method of measuring a PTT using the touch panel apparatus capable of measuring the PTT in a simple and convenient scheme.

Additional aspects will be set forth in part in the description which follows and, in part, will be apparent from the description, or may be learned by practice of the presented exemplary embodiments.

According to an aspect of an exemplary embodiment, there is provided a touch panel apparatus for measuring biosignals, the touch panel apparatus comprising a touch panel configured to detect a heartbeat-based signal from a first part of a target body; a pulse sensor configured to detect a pulse signal from a second part of the target body; and a processor configured to acquire a pulse transit time (PTT) based on the detected heartbeat-based signal and the detected pulse signal, wherein the touch panel apparatus contemporaneously detects the heartbeat-based signal and the pulse signal using the touch panel and the pulse sensor, respectively.

The touch panel and the pulse sensor may be located in different external surface portions of the touch panel apparatus.

The pulse sensor may be located at at least one position among positions of fingers holding the touch panel apparatus when the touch panel is in contact with the first part of the target body in a state in which the target body holds the touch panel apparatus.

The pulse sensor may be located in the external surface portion in a direction opposing the external surface portion in which the touch panel is located in the touch panel apparatus.

The pulse sensor may include a light-emitter and a light-receiver, wherein the light-emitter emits light having an optimum intensity based on a change in an optical characteristic of the second part of the target body according to the intensity of the light.

The touch panel apparatus may contemporaneously detect the heartbeat-based signal and the pulse signal in a state in which a height of the first part of the target body from the ground is substantially identical to a height of the second part of the target body from the ground.

The processor may eliminate noise of the heartbeat-based signal and the pulse signal, and detect a peak from each of the noise-eliminated heartbeat-based signal and the noise-eliminated pulse signal to acquire a representative value of a detected time difference between mutually corresponding peaks of the noise-eliminated heartbeat-based signal and the noise-eliminated pulse signal as the PTT.

The pulse sensor may automatically detect the pulse signal when the touch panel is switched from a mode in which a screen is manipulated according to a touch input signal to a mode in which the heartbeat-based signal is detected.

The touch panel may display biological information of the target body estimated from the PTT when the touch panel is switched from the mode in which the heartbeat-based signal is detected to the mode in which the screen is manipulated according to the touch input signal.

The touch panel apparatus may transmit at least one of the heartbeat-based signal, the pulse signal, and the PTT to a user terminal linked to the touch panel apparatus.

According to another aspect of an exemplary embodiment, there is provided a method of measuring a PTT using a touch panel apparatus, the method comprising detecting a heartbeat-based signal from a first part of a target body using a touch panel included in the touch panel apparatus; detecting a pulse signal from a second part of the target body using a pulse sensor included in the touch panel apparatus; and acquiring the PTT based on the detected heartbeat-based signal and the detected pulse signal, wherein the detecting of the heartbeat-based signal and the detecting of the pulse signal are contemporaneously performed in the touch panel apparatus.

The detecting of the heartbeat-based signal and the detecting of the pulse signal may include using the touch panel and the pulse sensor, respectively, that are located in different external surface portions of the touch panel apparatus.

The detecting of the pulse signal may include using the pulse sensor located at at least one position among positions of fingers holding the touch panel apparatus when the touch panel is in contact with the first part of the target body in a state in which the target body holds the touch panel apparatus.

The detecting of the pulse signal may include using the pulse sensor located in an external surface portion in a direction opposing an external surface portion in which the touch panel is located in the touch panel apparatus.

The detecting of the pulse signal may comprise emitting light having an optimum intensity based on a change in an optical characteristic of the second part of the target body according to the intensity of the light; and receiving light corresponding to a change in the second part of the target body as a response for the emitted light.

The heartbeat-based signal and the pulse signal may be contemporaneously detected in a state in which a height of the first part from the ground is substantially identical to a height of the second part from the ground.

The acquiring of the PTT may comprise eliminating noise from the heartbeat-based signal and the pulse signal; detecting a peak from each of the noise-eliminated heartbeat-based signal and the noise-eliminated pulse signal; and acquiring a representative value of a detected time difference between mutually corresponding peaks of the noise-eliminated heartbeat-based signal and the noise-eliminated pulse signal as the PTT.

The detecting of the pulse signal may comprise automatically detecting the pulse signal when the touch panel is switched from a mode in which a screen is manipulated according to a touch input signal to a mode in which the heartbeat-based signal is detected.

The method may further comprise displaying, by the touch panel, biological information of the target body estimated from the PTT when the touch panel is switched from the mode in which the heartbeat-based signal is detected to the mode in which the screen is manipulated according to the touch input signal.

According to another aspect of an exemplary embodiment, there is provided a non-transitory computer-readable recording medium recording a program for causing a computer to execute the method comprising detecting a heartbeat-based signal from a first part of a target body using a touch panel included in the touch panel apparatus; detecting a pulse signal from a second part of the target body using a pulse sensor included in the touch panel apparatus; and acquiring the PTT based on the detected heartbeat-based signal and the detected pulse signal, wherein the detecting of the heartbeat-based signal and the detecting of the pulse signal are contemporaneously performed in the touch panel apparatus.

According to another aspect of an exemplary embodiment, there is provided a touch panel apparatus comprising a touch panel provided on a first external surface of the touch panel apparatus and configured to detect, using the touch panel, a heartbeat-based signal from a first part of a target body; a pulse sensor provided on at least one second external surface of the touch panel apparatus that is different than the first external surface, the pulse sensor configured to detect a pulse signal from a second part of the target body; and a processor configured to acquire a pulse transit time (PTT) based on the detected heartbeat-based signal and the detected pulse signal.

The heartbeat-based signal and the pulse signal may be contemporaneously detected using the touch panel and the pulse sensor, respectively.

The heartbeat-based signal may be at least one of an electrocardiography (ECG) signal, an electromyography (EMG) signal, and an electroencephalography (EEG) signal.

The heartbeat-based signal may be detected based on a change in an electrostatic capacitance of the touch panel.

The pulse signal may be detected based on a change in light in the second part of the target body.

The pulse sensor may comprise a plurality of pulse sensors provided on the at least one second external surface.

The pulse sensor may be provided in a camera module of the touch panel apparatus.

The target body may be a body of a user, the first part may be a chest of the user, and the second part may be a finger of the user.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a diagram illustrating a touch panel apparatus according to an exemplary embodiment;
FIG. 2 is a diagram illustrating a position of a pulse sensor in the touch panel apparatus according to an exemplary embodiment;
FIG. 3 is a diagram illustrating a touch panel and the pulse sensor located in external surface portions of the touch panel apparatus according to an exemplary embodiment;
FIG. 4 is a diagram illustrating an operation of the pulse sensor of the touch panel apparatus according to an exemplary embodiment;
FIG. 5 is a diagram illustrating a process of measuring a pulse transit time (PTT) in the touch panel apparatus according to an exemplary embodiment;
FIG. 6 is a diagram illustrating heights of first and second parts of a target body from the ground when a heartbeat-based signal and a pulse signal are contemporaneously detected using the touch panel apparatus according to an exemplary embodiment;
FIG. 7 is a diagram illustrating a process of comparing the detected heartbeat-based signal with the detected pulse signal in the touch panel apparatus according to an exemplary embodiment;
FIG. 8 is a diagram illustrating a process of acquiring a PTT from peaks of the detected heartbeat-based signal and the pulse signal in the touch panel apparatus according to an exemplary embodiment;
FIG. 9 is a flowchart illustrating a method of measuring the PTT using the touch panel apparatus according to an exemplary embodiment; and
FIG. 10 is a detailed flowchart illustrating an operation of acquiring the PTT in the method of measuring the PTT illustrated in FIG. 9, using the touch panel apparatus according to an exemplary embodiment.

### DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and are not to be construed as being limited to the descriptions set forth herein. Accordingly, the exemplary embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

In the specification, terms "configure" or "include" should not necessarily be construed to indicate that the corresponding components or operations are all included, but rather that some of the components or operations may be omitted and other additional components or operations may be included.

It will be understood that, although the terms "first," "second," etc., may be used herein to describe various components, these elements should not be limited by these terms. The terms are used only for the purpose of distinguishing one component from another component.

In this specification, a biosignal includes a bioelectric signal, a bioimpedance signal, or the like, as a general term indicating a signal detected from the human body. The bioelectric signal is a signal of the form of a current or a voltage generated by a nerve cell or a muscle cell. For example, an electrocardiography (ECG) signal, an electromyography (EMG) signal, an electroencephalography (EEG) signal, or the like corresponds to the bioelectric signal. The bioimpedance signal is a signal according to a voltage drop generated by the impedance of body tissue after a current is applied to the tissue. The current may be predetermined. The bioimpedance signal may provide information about tissue structure, blood volume, blood distribution, etc. For example, body fat may be measured using the bioimpedance signal.

In this specification, the touch panel apparatus is an apparatus for allowing the user to input a signal by bringing a part of the user's body in contact with a screen of the touch panel apparatus. "Touch panel apparatus" is a generic term indicating all types of apparatuses to which a touch panel such as a touch screen or a touch pad is applied. The touch panel apparatus may be a mobile terminal such as a smartphone or a wearable device such as smartglasses or a smartwatch, but is not limited thereto. When a part of the user's body is in contact with a text character, an image, or the like displayed by a display equipped with the touch panel, the touch panel apparatus may find a user-selected item according to a contact screen position, process a command corresponding to the found item using a processor, and display user-desired information on the screen. The touch panel apparatus may be implemented in various schemes, but may be of a capacitive type in which a change in electrostatic capacitance is detected.

These exemplary embodiments relate to a touch panel apparatus for measuring biosignals and a method of measuring a PTT using the touch panel apparatus. In the following exemplary embodiments, detailed description of items well known to those skilled in the art will be omitted for concision of description.

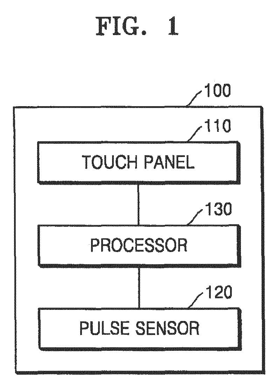

FIG. 1 is a diagram illustrating a touch panel apparatus 100 according to an exemplary embodiment. Those skilled in the art will readily understand that general-purpose components other than components illustrated in FIG. 1 may be further included.

The touch panel apparatus 100 may control an operation of the touch panel apparatus 100 according to the user's touch input signal, or may control the operation of the touch panel apparatus 100 to measure the user's biosignal. For this, the touch panel apparatus 100 may have two operation modes, including a manipulation mode and a measurement mode. The manipulation mode is a mode in which the screen of the touch panel apparatus 100 is operated based on the touch input signal, and the measurement mode is a mode in which the biosignal is measured based on the touch input signal. The touch panel apparatus 100 may have at least two operation modes including the manipulation mode and the measurement mode. The touch panel apparatus 100 may switch the mode based on a mode switching request from the user or based on the detected touch input signal. As another example, the touch panel apparatus 100 may recognize a hovering signal to operate in the measurement mode or the manipulation mode. In this example, the meaning of the touch may include the case in which the user directly touches the touch panel and the case in which the user generates the hovering signal through motion of his/her body on the touch panel.

The touch panel apparatus 100 may process and display a screen based on the user's touch input signal input to the screen in the manipulation mode. For example, when the user touches the screen of the touch panel apparatus 100 with two fingers to perform a pinch gesture, the touch panel apparatus 100 may detect the touch input signal corresponding to the user's pinch gesture to decrease or increase a screen size and display the decreased or increased screen. As another example, when the user performs the pinch gesture using the two fingers above the screen of the touch panel apparatus 100, the touch panel apparatus 100 may detect the hovering signal corresponding to the pinch gesture of the user to decrease or increase the screen size and display the decreased or increased screen.

The touch panel apparatus 100 may measure a biosignal based on the user's touch input signal input to the screen in the measurement mode. For example, when the user brings a part of his/her body in contact with the screen of the touch panel apparatus 100 and does not move for a given time, the touch panel apparatus 100 may detect a touch input signal corresponding to a change in the electric field for the part of the user's body in contact with the screen and measure the biosignal.

Referring to FIG. 1, the touch panel apparatus 100 includes a touch panel 110, a pulse sensor 120, and a processor 130. The processor 130 may be one or more microprocessors, one or more central processing units (CPUs), or one or more application specific integrated circuits (ASICs), or some combination of these components.

When the touch panel apparatus 100 operates in the measurement mode, the touch panel 110 can measure a biosignal based on the user's touch input signal. For example, when the touch panel apparatus 100 operates in the measurement mode according to the contact of a first part of a target body from which the biosignal is desired to be measured, the touch panel 110 may detect the heartbeat-based signal from the first part of the target body. The first part of the target body may be the chest or an ear, etc. The heartbeat-based signal detected from the first part of the target body may be an electrocardiography (ECG) signal or ballistocardiography (BCG) signal. For example, when the user wears clothing, the touch panel 110 may detect the heartbeat-based signal detected from the chest according to a change in an electric field. Accordingly, even when the touch panel 110 is separated from the chest without direct contact with the chest, the heartbeat-based signal may be detected.

When the touch panel apparatus 100 operates in the measurement mode, the pulse sensor 120 may detect a pulse signal from a second part of the target body. For example, when the touch panel apparatus 100 is in the measurement mode according to contact of the pulse sensor 120 with the second part of the target body from which a biosignal is desired to be measured, the pulse sensor 120 may detect the pulse signal from the second part of the target body. The second part of the target body may be a finger or wrist as an end part of the body. The pulse sensor 120 may be a photoplethysmography (PPG) sensor.

The touch panel apparatus 100 may contemporaneously detect the heartbeat-based signal and the pulse signal using the touch panel 110 and the pulse sensor 120. When the heartbeat-based signal and the pulse signal are contemporaneously detected, this indicates that another signal is detected while one signal is detected or both of two signals are concurrently detected at any given time. Signal detection start and end time points need not be the same for the heartbeat-based signal and the pulse signal, and detection times of the heartbeat-based signal and the pulse signal need not be the same as each other. Of course, the heartbeat-based signal and the pulse signal may be simultaneously detected. In addition, when a sampling rate is sufficiently high, the two signals may be detected in a time division scheme. In this case, the heartbeat-based signal and the pulse signal may be considered to be contemporaneously detected.

The touch panel 110 and the pulse sensor 120 may be linked to each other. When the operation mode of the touch panel apparatus 100 is switched to the measurement mode by any one of the touch panel 110 and the pulse sensor 120, the other may be prepared to measure the biosignal according to the measurement mode. For example, when the touch panel 110 is switched from the mode in which the screen is manipulated according to the touch input signal to the mode in which the heartbeat-based signal is detected, the pulse sensor 120 may be automatically switched to the mode in which the pulse signal may be detected. In contrast, when the touch panel 110 is switched from the mode in which the heartbeat-based signal is detected to the mode in which the screen is manipulated according to the touch input signal, the pulse sensor 120 may terminate the mode in which the pulse signal may be detected. The linkage between the touch panel 110 and the pulse sensor 120 may change according to the user's setting. That is, for example, the user may enable the linking or may disable the linking by changing a setting of the touch panel apparatus 100.

When the operation mode of the touch panel apparatus 100 is switched to the manipulation mode, the touch panel 110 may display biological information of the target body estimated from the PTT. The processor 130 may acquire the PTT from the signals detected in the manipulation mode and estimate the biological information of the target body from the PTT. The processor 130 may acquire the PTT based on the signal according to the heartbeat-based signal detected by the touch panel 110 and the pulse signal detected by the pulse sensor 120.

On the other hand, the touch panel apparatus 100 may transmit the bioelectric signal detected by the touch panel apparatus 100 or the biological information to a user terminal (not illustrated) linked to the touch panel apparatus 100. For example, the touch panel apparatus 100 may transmit at least one of the heartbeat-based signal detected by the touch panel 110, the pulse signal detected by the pulse sensor 120, and the PTT acquired by the processor 130 to the user terminal. The touch panel apparatus 100 may transfer the bioelectric signal to the user terminal by wireless or wired communication. Accordingly, the user may know the biosignal or the current situation through the user terminal linked to the touch panel apparatus 100 in real time while measuring the biosignal using the touch panel apparatus 100. After the measurement of the biosignal is completed using the touch panel apparatus 100, the user may transmit a measurement result including a biosignal detected by the user terminal linked to the touch panel apparatus 100. The user terminal may include various types of devices such as a mobile device, a wearable device, and a stationary device.

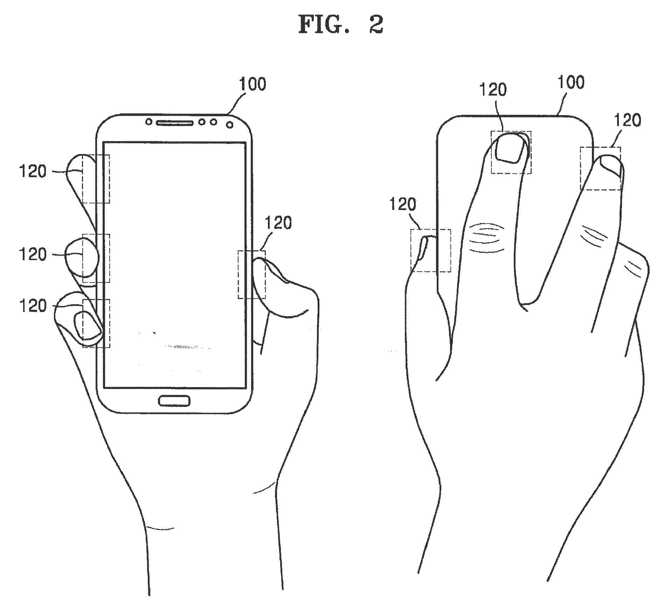

FIG. 2 is a diagram illustrating a position of the pulse sensor 120 in the touch panel apparatus 100 according to an exemplary embodiment.

At least one pulse sensor 120 may be located in the touch panel apparatus 100. In a case in which there are a plurality of pulse sensors 120, the pulse sensors 120 may be located in different external surfaces of the touch panel apparatus 100 or located in the same external surface of the touch panel apparatus 100. FIG. 2 shows an example in which a plurality of pulse sensors 120 are provided. However, this is only an example, and in some exemplary embodiments only one pulse sensor 120 may be provided, and in other exemplary embodiments, a different number of pulse sensors 120 may be provided other than the number shown in FIG. 2.

The pulse sensor 120 and the touch panel 110 may be located in different external surface portions of the touch panel apparatus 100. For example, when the touch panel 110 is located in a front surface of the touch panel apparatus 100, the pulse sensor 120 may be located in any one of an upper surface, a lower surface, a left surface, a right surface, and a rear surface of the touch panel apparatus 100. As described above, in the case in which a plurality of pulse sensors 120 are provided, the pulse sensors 120 may be provided in more than one of the upper surface, the lower surface, the left surface, the right surface, and the rear surface. Because the external surface portion of the touch panel apparatus 100 in which the touch panel 110 is located may not be in contact with the second part of the target body when the touch panel 110 is in contact with the first part of the target body, it is advantageous for the pulse sensor 120 to be located in a different external surface portion from an external surface portion of the touch panel apparatus 100 in which the touch panel 110 is located.

Referring to FIG. 2, an example is illustrated in which a portion in which the pulse sensor 120 may be located in the touch panel apparatus 100 is indicated by a dotted-line box. The location(s) of the pulse sensor 120 is not limited thereto.

As illustrated in FIG. 2, the pulse sensor 120 may be located at at least one of positions of fingers holding the touch panel apparatus 100 when the user, i.e., the target body, brings the touch panel 110 in contact with the first part of the target body (e.g., the user's body) while holding the touch panel apparatus 100. When the touch panel apparatus 100 is held so that the user may bring the touch panel 110 in contact with the first part of the target body, the pulse sensor 120 may be located in one or more parts of the touch panel apparatus 100 that the user's finger is capable of reaching and touching. For example, the positions of the pulse sensors 120 may be set based on experimental data indicating positions in which users are likely to place fingers when holding the touch panel apparatus 100 to the first part of the user's body.

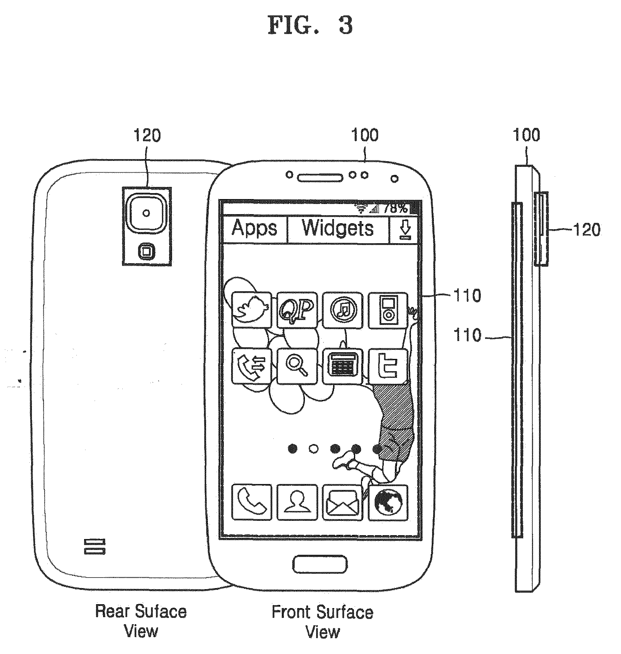

FIG. 3 is a diagram illustrating the touch panel 110 and the pulse sensor 120 located in external surface portions of the touch panel apparatus 100 according to an exemplary embodiment.

The pulse sensor 120 may comprise a light-emitter configured to radiate light to the second part of the target body and a light-receiver configured to receive light reflected from the second part of the target body. As illustrated in FIG. 3, the pulse sensor 120 may be implemented using an illumination device (e.g., a flash component of a camera module) provided in the touch panel apparatus 100 and an image sensor of a camera module.

If the pulse sensor 120 is located at a position as illustrated in FIG. 3, an index finger of the target body may be naturally located at positions of the camera module and the illumination device when the target body (e.g., the user) holds the touch panel apparatus 100 in order to bring the touch panel 110 in contact with the chest of the target body. In this case, the pulse sensor 120 is located in an external surface portion in a direction opposing the external surface portion in which the touch panel 110 is located in the touch panel apparatus 100. In other words, when the external surface portion in which the touch panel 110 is located in the touch panel apparatus 100 is referred to as a front surface portion, the pulse sensor 120 may be located in a rear surface portion.

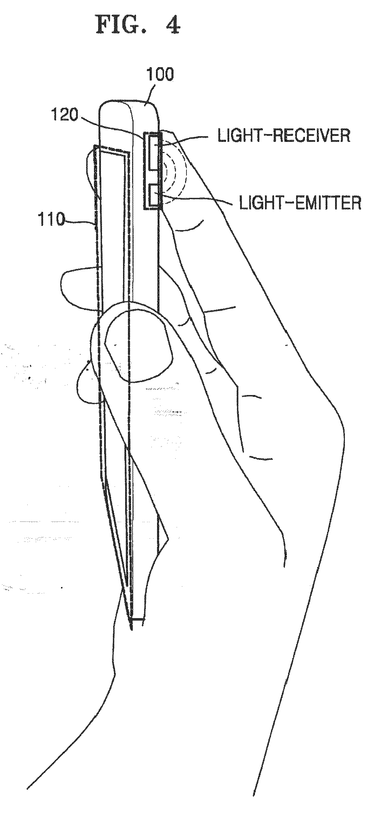

FIG. 4 is a diagram illustrating an operation of the pulse sensor 120 of the touch panel apparatus 100 according to an exemplary embodiment.

The pulse is a pulsating waveform shown while blood is discharged from the heart and may be measured through a change in a blood flow amount due to relaxation and contraction actions of the heart and a volume change in a blood vessel according to the change in the blood flow. For example, the pulse sensor 120 may measure a pulse signal by measuring an amount of blood flowing through the blood vessel using an optical characteristic of biological tissue.

The pulse sensor 120 may include a light-emitter and a light-receiver. The pulse sensor 120 may detect, using light, characteristics of light reflectance, light absorbance, and/or light transmittance, etc. of the biological tissue shown when the volume of the blood vessel changes and measure a pulse through a change in the characteristics.

The light-emitter of the pulse sensor 120 may emit light having an optimum intensity based on a change in the optical characteristics of the second part of the target body according to the intensity of the light.

The light-receiver of the pulse sensor 120 may receive light reflected from the biological tissue.

As illustrated in FIG. 4, the second part (e.g., a finger) of the target body (e.g., the user's body) may be in contact with the pulse sensor 120 when the user holds the touch panel apparatus 100 in one hand. The light-emitter may radiate light to the second part of the target body and the light-receiver may receive light reflected from the second part of the target so that the pulse sensor 120 measures the pulse signal when the contact of the second part of the target body is detected. While the user maintains this state, the touch panel apparatus 100 may be located so that the touch panel 110 is in contact with the first part (e.g., the chest) of the target body (e.g., the user's body). When the touch panel 110 is in contact with the first part of the target body, the touch panel 110 may detect the heartbeat-based signal from the first part of the target body.

That is, through a simple action in which the user holds the touch panel apparatus 100 in one hand and holds the touch panel 110 of the touch panel apparatus 100 on his/her chest, it is possible to measure the PTT based on a heartbeat-based signal detected from the heart and a pulse signal detected from the finger.

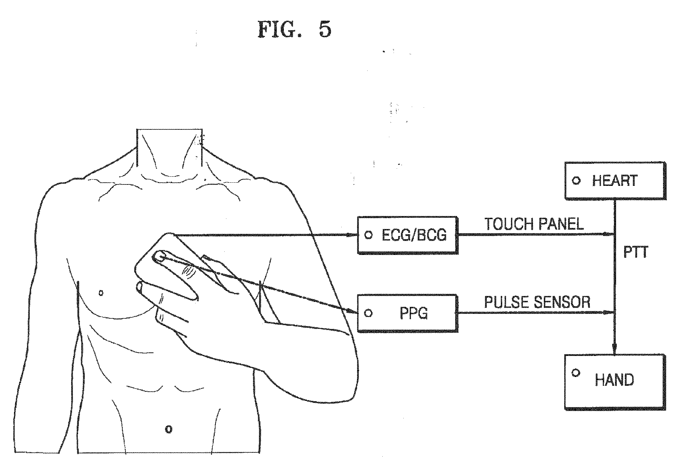

FIG. 5 is a diagram illustrating a process of measuring a PTT in the touch panel apparatus 100 according to an exemplary embodiment.

As illustrated in FIG. 5, the touch panel apparatus 100 may measure the PTT when the user holds the touch panel apparatus 100 in one hand and brings the touch panel 110 in contact with his/her chest.

The touch panel 110 of the touch panel apparatus 100 may detect the heartbeat-based signal such as the ECG or BCG from the first part (e.g., the chest) of the target body (e.g., the body of the user). The pulse sensor 120 of the touch panel apparatus 100 may detect the pulse signal from the second part (e.g., the finger) of the target body (e.g., the body of the user). The touch panel apparatus 100 may acquire the PTT from signals measured from different parts of the target body.

As illustrated in FIG. 5, the user may measure the PTT after only holding the touch panel apparatus 100 in one hand to hold the touch panel apparatus 100 on his/her chest because the touch panel apparatus 100 has a structure and form in which the heartbeat-based signal and the pulse signal may be contemporaneously detected. The touch panel apparatus 100 may detect the ECG or BCG using the touch panel 110 capable of measuring the biosignal and detect the pulse signal using the pulse sensor 120 provided in the touch panel apparatus 100. In other words, the touch panel apparatus 100 may detect the heartbeat-based signal without providing a separate sensor for detecting the ECG or BCG in the chest of the target body and may detect the pulse signal using the light-emitter and the light-receiver provided in the touch panel apparatus 100. The light-emitter and the light-receiver constituting the pulse sensor 120 may be replaced in some exemplary embodiments with the illumination device of the touch panel apparatus 100 and the image sensor of the camera module, respectively.

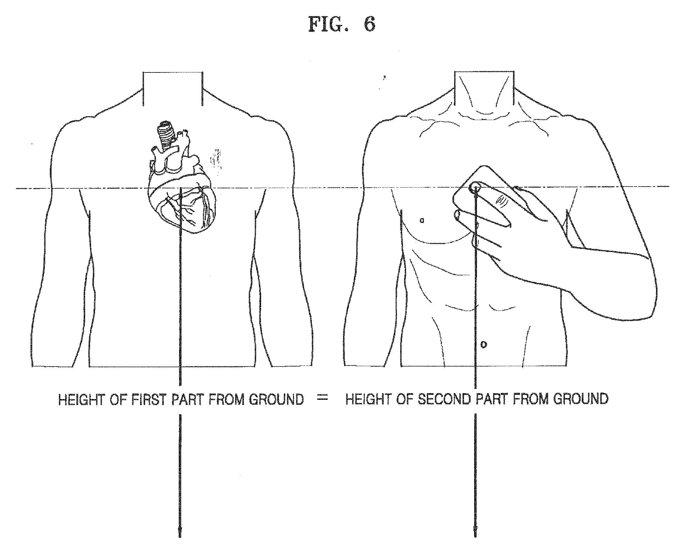

FIG. 6 is a diagram illustrating heights of first and second parts of a target body from the ground when a heartbeat-based signal and a pulse signal are contemporaneously detected using the touch panel apparatus 100 according to an exemplary embodiment.

As illustrated in FIG. 6, it may be seen that the height of the chest of the user corresponding to the first part of the target body from the ground is substantially identical to the height of the finger corresponding to the second part of the target body from the ground when the user holds the touch panel apparatus 100 in one hand and holds the touch panel apparatus 100 on his/her chest to measure the PTT. In other words, by this configuration, the height of the heart is substantially identical to the height of the finger as shown in FIG. 6.

The pulse sensor 120 may be arranged in a part that the user's finger can reach so that the user may naturally measure a pulse signal when the user holds the touch panel apparatus 100 in one hand. The part that the user's finger can reach may be determined experimentally based, for example, on an average position of users' fingers when users hold the touch panel apparatus 100 in one hand. When the user holds the touch panel 110 on his/her heart chest while holding the touch panel apparatus 100, the pulse signal may be detected through the pulse sensor 120 and the heartbeat-based signal may also be detected through the touch panel 110. Because the hand holding the touch panel apparatus 100, i.e., the hand from which the pulse signal is detected through the pulse sensor 120, directly moves to the chest, the height of the finger from the ground may be substantially identical to the height of the heart from the ground.

When the height of the first part of the target body from which the heartbeat-based signal is desired to be detected is substantially identical to the height of the second part of the target body from which the pulse signal is desired to be detected, it is possible to minimize an influence of a hydrostatic pressure on the second part of the target body.

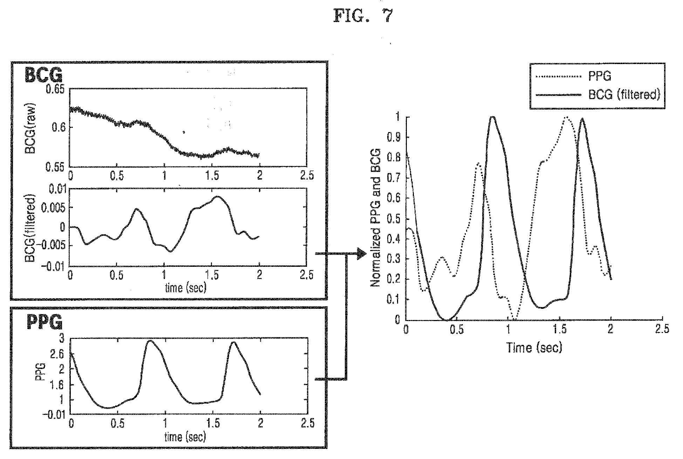

FIG. 7 is a diagram illustrating a process of comparing the detected heartbeat-based signal with the detected pulse signal in the touch panel apparatus 100 according to an exemplary embodiment.

The processor 130 of the touch panel apparatus 100 may compare the heartbeat-based signal such as the ECG or BCG signal detected by the touch panel 110 with the pulse signal detected by the pulse sensor 120 in order to acquire the PTT.

Before the two signals are compared, the processor 130 may eliminate noise from each of the two signals received from the touch panel 110 and the pulse sensor 120. In other words, the processor 130 may eliminate noise from each of the heartbeat-based signal received from the touch panel 110 and the pulse signal received from the pulse sensor 120 through, for example, a band-pass filter.

Referring to FIG. 7, it may be seen that BCG raw data (BCG(raw)) detected by the touch panel 110 and a BCG signal (BCG(filtered)) obtained by eliminating noise through a filtering process on the BCG raw data are displayed in an upper left portion of FIG. 7 and the pulse signal (PPG) obtained by eliminating noise through a filtering process is displayed in a lower left portion of FIG. 7. The BCG filtered signal and the pulse signal (PPG) may be compared by comparing peaks of the signals in a state in which the two filtered signals are arranged on a time axis.

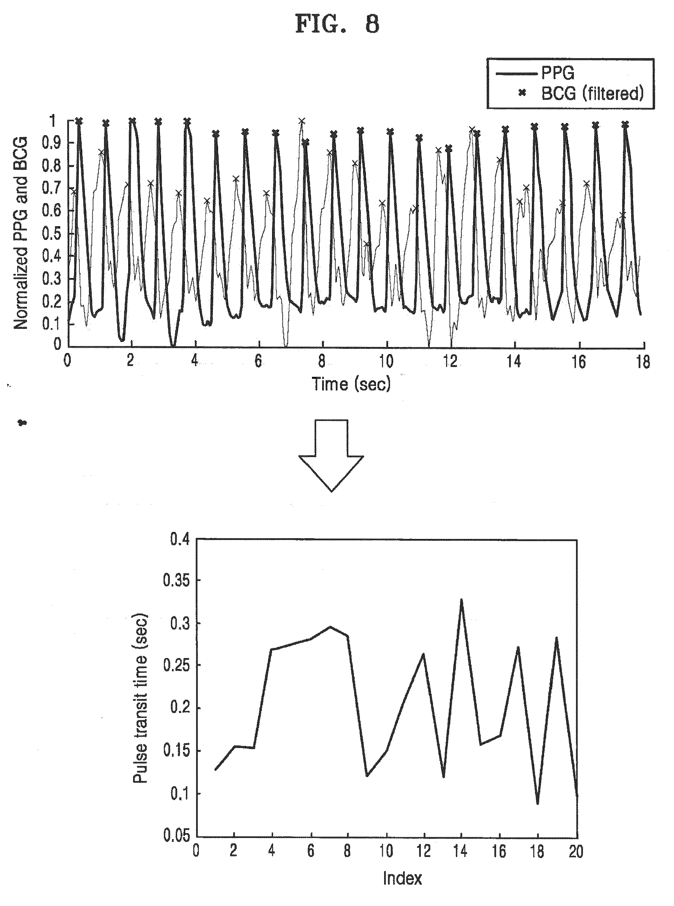

FIG. 8 is a diagram illustrating a process of acquiring a PTT from peaks of the detected heartbeat-based signal and the pulse signal in the touch panel apparatus according to an exemplary embodiment.

The processor 130 of the touch panel apparatus 100 may detect the peaks after noise is eliminated from the heartbeat-based signal such as the ECG or BCG signal detected by the touch panel 110 and the pulse signal detected by the pulse sensor 120. As illustrated on the top in FIG. 8, portions corresponding to peaks of each of the BCG signal and the pulse signal are indicated by 'X.'

The processor 130 of the touch panel apparatus 100 may detect peak parts of the BCG signal and the pulse signal, check corresponding peaks between the two signals, and obtain a difference between peak detection times. The processor 130 may acquire a representative value of a difference between times at which the peaks are detected from the two signals as the PTT. For example, the PTT may be an average or a mean value of the difference between the times at which the corresponding peaks of the two signals are detected. The bottom of FIG. 8 is a graph showing the PTT based on the peaks in the top of FIG. 8.

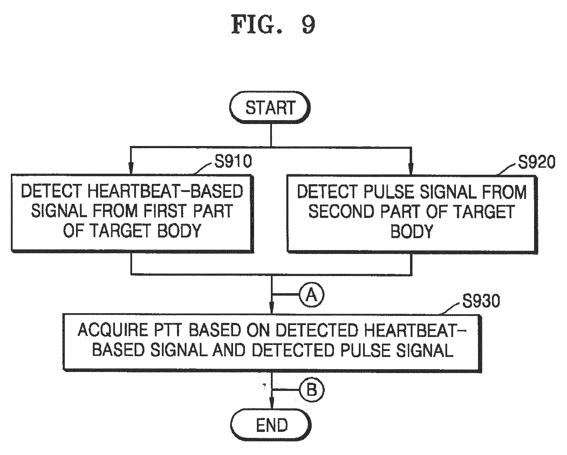

FIG. 9 is a flowchart illustrating a method of measuring the PTT using the touch panel apparatus according to an exemplary embodiment.

Even if not explicitly stated below, the above description of the touch panel apparatus 100 may also be applied to the method of measuring the PTT using the touch panel apparatus 100.

In operation 910, the touch panel apparatus 100 may detect a heartbeat-based signal from the first part of the target body using the touch panel 110 included in the touch panel apparatus 100. The touch panel apparatus 100 may transmit the heartbeat-based signal detected by the touch panel apparatus 100 to the user terminal linked to the touch panel apparatus 100 to display the detected heartbeat-based signal on the user terminal linked to the touch panel apparatus 100.

In operation 920, the touch panel apparatus 100 may detect the pulse signal from the second part of the target body using the pulse sensor 120 included in the touch panel apparatus 100. The touch panel apparatus 100 may transmit the pulse signal detected by the touch panel apparatus 100 to the user terminal linked to the touch panel apparatus 100 to display the detected pulse signal on the user terminal linked to the touch panel apparatus 100.

When the touch panel 110 is switched from the mode in which the screen is manipulated according to the touch input signal to the mode in which the heartbeat-based signal is detected, the pulse signal may also be automatically detected.

To detect the heartbeat-based signal and the pulse signal, the touch panel apparatus 100 may use the touch panel 110 and the pulse sensor 120 located in different external surface portions of the touch panel apparatus 100.

When the touch panel 110 is in contact with the first part in a state in which the target body holds the touch panel apparatus 100, it is possible to use a pulse sensor located in at least one position among positions of fingers holding the touch panel apparatus 100. The pulse sensor 120 may be located in an external surface portion in a direction opposing an external surface portion in which the touch panel 110 is located in the touch panel apparatus 100.

The pulse sensor 120 may emit light having an optimum intensity based on a change in optical characteristics of the second part according to the intensity of light and receive light corresponding to a change in the second part as a response to the emitted light.

Operation 910 of detecting the heartbeat-based signal and operation 920 of detecting the pulse signal may be contemporaneously performed. When the operation of detecting the heartbeat-based signal and the operation of detecting the pulse signal along with the heartbeat-based signal are contemporaneously performed, this indicates that another signal is detected while one signal is detected or both of two signals are concurrently detected at any given time. Signal detection start and end time points need not be the same as each other and detection times of the heartbeat-based signal and the pulse signal need not be the same as each other. Of course, the heartbeat-based signal and the pulse signal may be simultaneously detected. In addition, when a sampling rate is sufficiently high, the heartbeat-based signal and the pulse signal may be detected in a time division scheme. In this case, the heartbeat-based signal and the pulse signal may be considered to be contemporaneously detected.

Moreover, it is possible to contemporaneously detect the heartbeat-based signal and the pulse signal in a state in which the height of the first part of the target body from the ground is substantially identical to the height of the second part of the target body from the ground.

In operation 930, the touch panel apparatus 100 may acquire the PTT based on the heartbeat-based signal and the pulse signal.

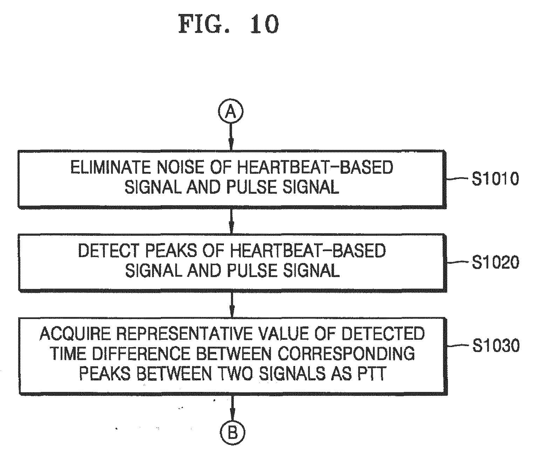

FIG. 10 is a detailed flowchart illustrating the operation of acquiring the PTT in the method of measuring the PTT using the touch panel apparatus according to an exemplary embodiment.

In operation 1010, the touch panel apparatus 100 may eliminate noise of a heartbeat-based signal and a pulse signal.

In operation 1020, the touch panel apparatus 100 may detect a peak of each of the heartbeat-based signal and the pulse signal. For example, the touch panel apparatus 100 may detect peaks of each signal as shown in FIG. 8.

In operation 1030, the touch panel apparatus 100 may acquire a representative value of a time difference detected between corresponding peaks between the heartbeat-based signal and the pulse signal as the PTT. To display the acquired PTT to the user terminal linked to the touch panel apparatus 100, the touch panel apparatus 100 may transmit the PTT acquired by the touch panel apparatus 100 to the user terminal linked to the touch panel apparatus 100. The touch panel apparatus 100 may transmit biological information of the target body estimated from the PTT to the user terminal linked to the touch panel apparatus 100.

The touch panel apparatus 100 may display the biological information of the target body estimated from the PTT when the touch panel 110 is switched from the mode in which the heartbeat-based signal is detected to the mode in which the screen is manipulated according to a touch input signal. The biological information of the target body capable of being estimated from the PTT may be blood pressure, stress, an exercise effect, or the like.

The above-described method of measuring a PTT using the touch panel apparatus 100 may be provided as a program executable in a computer. The program may be implemented in a general-purpose digital computer that operates the program using a non-transitory computer-readable recording medium. The non-transitory computer-readable medium includes a storage medium such as a magnetic storage medium (for example, a ROM, a floppy disk, or a hard disk) or an optical readable medium (for example, a CD-ROM or a digital versatile disc (DVD)).

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope as defined by the following claims.

## Claims

1. A touch panel apparatus for measuring biosignals, the touch panel apparatus comprising:
a touch panel configured to detect a heartbeat-based signal from a first part of a target body;
a pulse sensor configured to detect a pulse signal from a second part of the target body; and
a processor configured to acquire a pulse transit time, PTT, based on the detected heartbeat-based signal and the detected pulse signal,
wherein the touch panel apparatus contemporaneously detects the heartbeat-based signal and the pulse signal using the touch panel and the pulse sensor, respectively.

2. The touch panel apparatus according to claim 1, wherein the touch panel and the pulse sensor are located in different external surface portions of the touch panel apparatus.

3. The touch panel apparatus according to claim 2, wherein the pulse sensor is located at at least one position among positions of fingers holding the touch panel apparatus when the touch panel is in contact with the first part of the target body in a state in which the target body holds the touch panel apparatus, or wherein the pulse sensor is located in the external surface portion in a direction opposing the external surface portion in which the touch panel is located in the touch panel apparatus.

4. The touch panel apparatus according to one of claims 1 to 3,
wherein the pulse sensor includes a light-emitter and a light-receiver, and
wherein the light-emitter emits light having an optimum intensity based on a change in an optical characteristic of the second part of the target body according to the intensity of the light.

5. The touch panel apparatus according to one of claims 1 to 4, wherein the touch panel apparatus contemporaneously detects the heartbeat-based signal and the pulse signal in a state in which a height of the first part of the target body from the ground is substantially identical to a height of the second part of the target body from the ground, or
wherein the touch panel displays biological information of the target body estimated from the PTT when the touch panel is switched from the mode in which the heartbeat-based signal is detected to the mode in which the screen is manipulated according to the touch input signal, or
wherein the touch panel apparatus transmits at least one of the heartbeat-based signal, the pulse signal, and the PTT to a user terminal linked to the touch panel apparatus.

6. The touch panel apparatus according to one of claims 1 to 5, wherein the processor eliminates noise of the heartbeat-based signal and the pulse signal, and detects a peak from each of the noise-eliminated heartbeat-based signal and the noise-eliminated pulse signal to acquire a representative value of a detected time difference between mutually corresponding peaks of the noise-eliminated heartbeat-based signal and the noise-eliminated pulse signal as the PTT.

7. The touch panel apparatus according to one of claims 1 to 6, wherein the pulse sensor automatically detects the pulse signal when the touch panel is switched from a mode in which a screen is manipulated according to a touch input signal to a mode in which the heartbeat-based signal is detected.

8. A method of measuring a PTT using a touch panel apparatus, the method comprising:
detecting a heartbeat-based signal from a first part of a target body using a touch panel included in the touch panel apparatus;
detecting a pulse signal from a second part of the target body using a pulse sensor included in the touch panel apparatus; and
acquiring the PTT based on the detected heartbeat-based signal and the detected pulse signal,
wherein the detecting of the heartbeat-based signal and the detecting of the pulse signal are contemporaneously performed in the touch panel apparatus.

9. The method according to claim 8, wherein the detecting of the heartbeat-based signal and the detecting of the pulse signal include using the touch panel and the pulse sensor, respectively, that are located in different external surface portions of the touch panel apparatus.

10. The method according to claim 9, wherein the detecting of the pulse signal includes using the pulse sensor located at at least one position among positions of fingers holding the touch panel apparatus when the touch panel is in contact with the first part of the target body in a state in which the target body holds the touch panel apparatus, or
wherein the detecting of the pulse signal includes using the pulse sensor located in an external surface portion in a direction opposing an external surface portion in which the touch panel is located in the touch panel apparatus.

11. The method according to one of claims 8 to 10, wherein the detecting of the pulse signal comprises: emitting light having an optimum intensity based on a change in an optical characteristic of the second part of the target body according to the intensity of the light; and receiving light corresponding to a change in the second part of the target body as a response for the emitted light, or
wherein the detecting of the pulse signal comprises automatically detecting the pulse signal when the touch panel is switched from a mode in which a screen is manipulated according to a touch input signal to a mode in which the heartbeat-based signal is detected.

12. The method according to one of claims 8 to 11, wherein the heartbeat-based signal and the pulse signal are contemporaneously detected in a state in which a height of the first part from the ground is substantially identical to a height of the second part from the ground.

13. The method according to one of claims 8 to 12, wherein the acquiring of the PTT comprises:
eliminating noise from the heartbeat-based signal and the pulse signal;
detecting a peak from each of the noise-eliminated heartbeat-based signal and the noise-eliminated pulse signal; and
acquiring a representative value of a detected time difference between mutually corresponding peaks of the noise-eliminated heartbeat-based signal and the noise-eliminated pulse signal as the PTT.

14. The method according to one of claims 8 to 13, further comprising:
displaying, by the touch panel, biological information of the target body estimated from the PTT when the touch panel is switched from the mode in which the heartbeat-based signal is detected to the mode in which the screen is manipulated according to the touch input signal.

15. A non-transitory computer-readable recording medium recording a program for causing a computer to execute the method comprising:
detecting, using a touch panel, a heartbeat-based signal from a first part of a target body using a touch panel included in the touch panel apparatus; said touch panel provided on a first external surface of a touch panel apparatus;
detecting a pulse signal from a second part of the target body using a pulse sensor included in the touch panel apparatus; said pulse sensor provided on at least one second external surface of the touch panel apparatus that is different than the first external surface; and
acquiring a pulse transit time, PTT, based on the detected heartbeat-based signal and the detected pulse signal.
